# EUROPEAN PATENT APPLICATION

(11) **EP 4 414 007 A1**
(43) Date of publication of application: **14.08.2024**
(21) Application number: 22878344.5
(22) Date of filing: 26.09.2022
(51) Int. Cl.: A61M 5/32

(54) **INJECTION NEEDLE**

(30) Priority: 07.10.2021 JP 2021165236
(71) Applicant: TERUMO Kabushiki Kaisha, Tokyo 151-0072 (JP)
(72) Inventor: TANAKA, Ryo, Ashigarakami-gun, Kanagawa 259-0151 (JP); IWASE, Yoichiro, Ashigarakami-gun, Kanagawa 259-0151 (JP); YABE, Hisao, Ashigarakami-gun, Kanagawa 259-0151 (JP)
(74) Representative: Casalonga
(86) International application number: PCT/JP2022/035572
(87) International publication number: WO 2023/058473

(57) **Abstract**

There is provided an injection needle that perpendicularly punctures a target site such as an intramucosal or intradermal site and injects a medicinal solution into the target site. The injection needle includes: a bevel-needle-shaped needle body having a hollow portion through which the medicinal solution is passable; a needle body blade surface which is formed at a distal portion of the needle body and enables a puncture of the target site; and a passage hole which is formed at the distal portion and through which the medicinal solution passes. The passage hole is biased toward a side of a distal end of the needle body blade surface. A length of the needle body in an axial direction from an edge portion of an opening edge of the passage hole to the distal end of the needle body blade surface is 0.1 mm or longer and shorter than 2.0 mm.

## Description

### BACKGROUND

### Technical Field

The present invention relates to an injection needle.

### Background Art

A medical device including a first needle and a second needle accommodated in a lumen of the first needle is known (see Patent Literature 1). The second needle is disposed eccentrically outward in a radial direction from the axial center of the first needle. The second needle is slidable while being guided by an inner wall of the lumen of the first needle. At least a distal end of a lumen of the second needle is eccentrically disposed on a side opposite to a side where the second needle is eccentrically disposed such that the distal end thereof is close to the axial center of the first needle. In this medical device, after the first needle punctures a blood vessel, the second needle is exposed from the first needle, and a biodegradable material is injected from the lumen distal end of the second needle. By disposing the biodegradable material on an outer circumference of the blood vessel, extravasation of a medicinal solution is prevented in performing an intravenous injection.

### Citation List

### Patent Literature

Patent Literature 1: JP 6765990 B2

### SUMMARY OF INVENTION

### Technical Problem

Incidentally, in order to inject a medicinal solution into a very thin target site such as an intramucosal or intradermal site, an injection needle may be used by puncturing a surface of the target site perpendicularly. A blade surface of a general-purpose injection needle has an obliquely inclined shape (bevel needle shape). Therefore, a distal end of the blade surface pierces beyond the target site into underlying tissue under the target site, and there is a possibility of a leakage of the medicinal solution into the underlying tissue. In addition, although the distal end of the blade surface does not pierce beyond the target site, a lumen distal end is located above the target site, and there is a possibility of a leakage of the medicinal solution to the surface of the target site. Further, in the case of a so-called obtuse needle with a blade surface having a short length (bevel length), puncture resistance is high, and there is a possibility that it is difficult to achieve a puncture of a target site.

Even when a medical device disclosed in JP 6765990 B2 is used by perpendicularly puncturing the surface of the target site, the lumen distal end of the second needle is located close to the axial center of the first needle. Therefore, a problem such as a leakage of a medicinal solution into underlying tissue described above arises.

In this respect, an object of the present invention is to provide an injection needle which enables a puncture of a target site such as an intramucosal or intradermal site without increasing puncture resistance and enables injection of a medicinal solution into the target site without causing a leakage of the medicinal solution.

### Solution to Problem

An injection needle according to the present invention is an injection needle that perpendicularly punctures a surface of a target site such as an intramucosal or intradermal site and injects a medicinal solution into the target site. The injection needle includes a bevel-needle-shaped needle body having a hollow portion through which the medicinal solution is passable; a needle body blade surface that is formed at a distal portion of the needle body and enables a puncture of the target site; and a passage hole which is formed at the distal portion of the needle body and through which the medicinal solution from the hollow portion passes. The passage hole is disposed biasedly toward a side on which a distal end of the needle body blade surface is positioned. A length in an axial direction of the needle body from an edge portion of an opening edge of the passage hole on a side opposite to a biased side of the opening edge to the distal end of the needle body blade surface is set to 0.1 mm or longer and shorter than 2.0 mm.

### Advantageous Effects of Invention

According to the injection needle of the present invention, the passage hole is disposed biasedly toward a side on which a distal end of the needle body blade surface is positioned. Further, the length of the needle body in the axial direction from the edge portion of the passage hole to the distal end of the needle body blade surface is set to 0.1 mm or longer and shorter than 2.0 mm. As compared with the case where the passage hole is disposed at the position of the axial center of the bevel-needle-shaped needle body, it is possible to reduce a likelihood that the distal end of the needle body blade surface will pierce beyond the target site into underlying tissue under the target site. This makes it possible to prevent the medicinal solution from leaking into the underlying tissue. In addition, it is possible to reduce a likelihood that the passage hole will be positioned above the target site. This makes it possible to prevent the medicinal solution from leaking to the surface of the target site. Further, since the needle body with the needle body blade surface having a relatively long length (bevel length) can be used, puncture resistance does not increase, and it is possible to reliably achieve a puncture of a target site. Hence, the injection needle can puncture a target site having a thickness dimension of more than 0.1 mm and 2.0 mm or less without increasing puncture resistance and can inject the medicinal solution into the target site without causing a leakage of the medicinal solution.

### BRIEF DESCRIPTION OF DRAWINGS

FIG. 1 is a front view illustrating an injection needle assembly to which an injection needle of the present invention is applied;
FIG. 2 is a schematic view illustrating a state where an injection needle of Embodiment 1 punctures a target site;
FIGS. 3A and 3B are a perspective view and a top view illustrating the injection needle of Embodiment 1;
FIGS. 4A and 4B are a front view and a side view illustrating the injection needle of Embodiment 1;
FIG. 5 is a graph illustrating an experimental result of puncture resistance of the injection needle;
FIGS. 6A, 6B, and 6C are a longitudinal-sectional view illustrating an injection needle of Embodiment 2, a cross-sectional view along line 6B-6B of FIG. 6A, and a top view of the injection needle;
FIGS. 7A, 7B, and 7C are a longitudinal-sectional view illustrating an injection needle of Embodiment 3, a cross-sectional view along line 7B-7B of FIG. 7A, and a top view of the injection needle;
FIGS. 8A, 8B, and 8C are schematic views illustrating a procedure for forming the injection needle of Embodiment 3;
FIGS. 9A, 9B, and 9C are a top view, a front view, and a side view illustrating an injection needle of Embodiment 4;
FIGS. 10A, 10B, and 10C are schematic views illustrating a procedure for forming the injection needle of Embodiment 4; and
FIGS. 11A and 11B are schematic views illustrating a procedure for forming an injection needle of Embodiment 5.

### DETAILED DESCRIPTION

Hereinafter, embodiments of the present invention will be described with reference to the accompanying drawings. In the description of the drawings, the same elements are denoted by the same reference numerals, and redundant description will be omitted. In addition, dimensional ratios of the drawings are exaggerated for illustration purpose and may differ from actual ratios.

In the present specification, a side for puncturing a target site 100 is referred to as a "distal end" or a "distal end side", and the opposite side is referred to as a "proximal end" or a "proximal end side". In addition, a portion indicating a certain range including the distal end and a surrounding portion thereof is referred to as a "distal portion". In addition, a direction in which a needle body 30 of an injection needle extends is referred to as an "axial direction".

### (Embodiment 1)

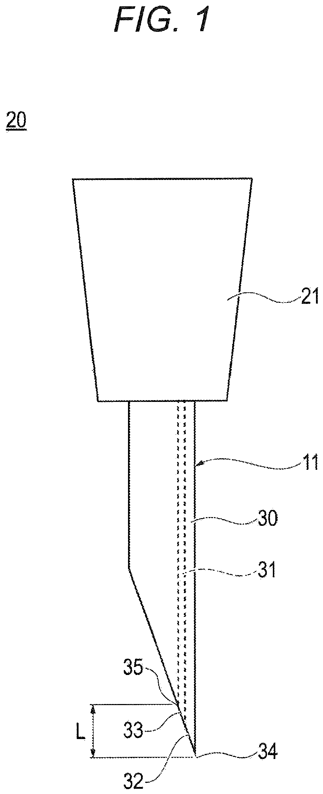

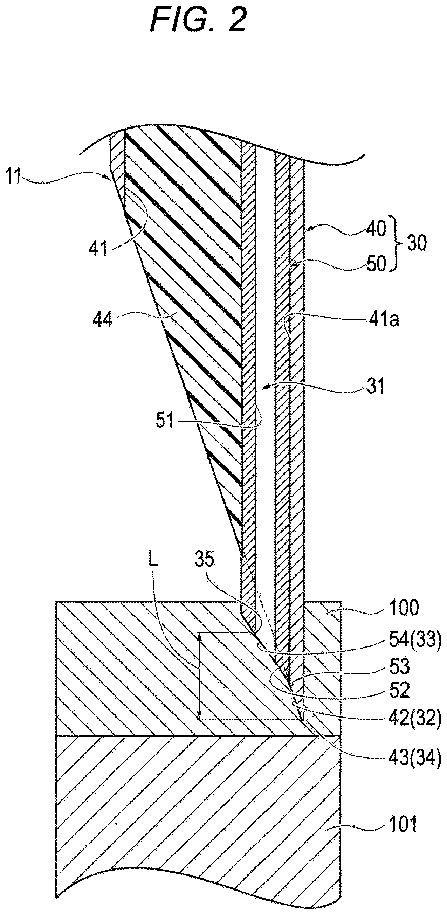

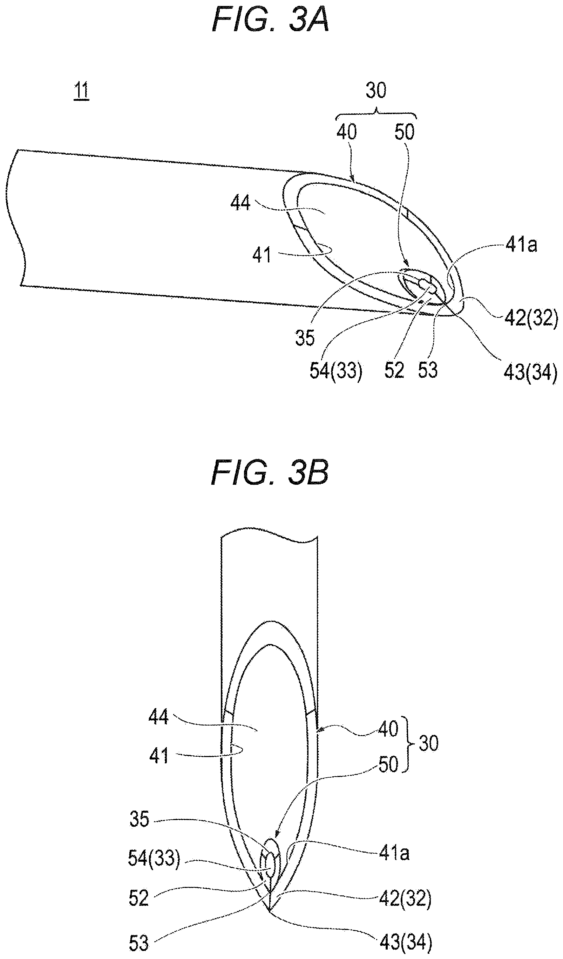

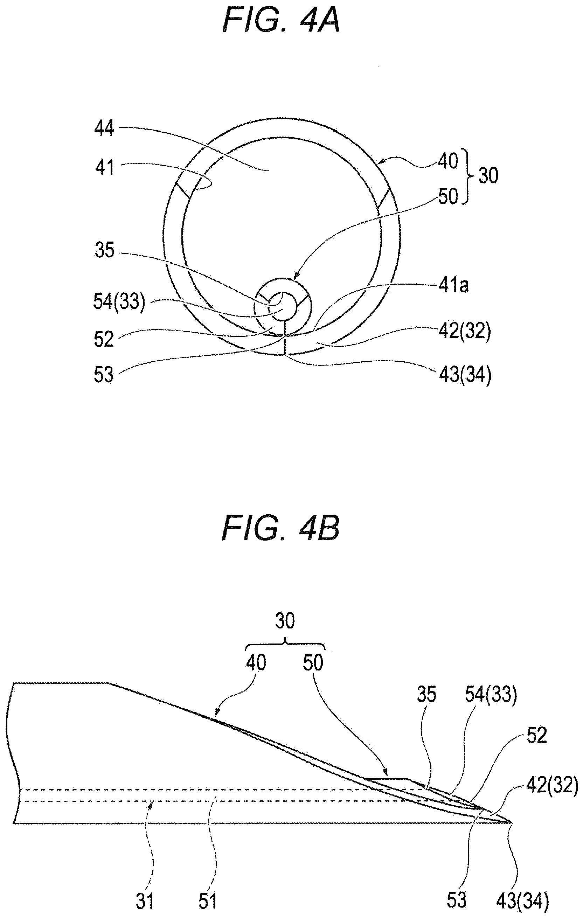

FIG. 1 is a front view illustrating an injection needle assembly 20 to which an injection needle 11 of the present invention is applied. FIG. 2 is a schematic view illustrating a state where the injection needle 11 of Embodiment 1 punctures a target site 100. FIGS. 3A and 3B are a perspective view and a top view illustrating the injection needle 11 of Embodiment 1. FIGS. 4A and 4B are a front view and a side view illustrating the injection needle 11 of Embodiment 1.

As illustrated in FIG. 1, the injection needle assembly 20 includes the injection needle 11 and a hub 21 that holds a proximal end of the injection needle 11. A syringe (not illustrated) is connected to a proximal end of the hub 21.

As illustrated in FIG. 2, the injection needle 11 is used to perpendicularly puncture a surface of the target site 100 such as an intramucosal or intradermal site and inject a medicinal solution into the target site 100. In the present specification, "perpendicular" means not only a case of a puncture of the surface of the target site 100 at an angle of 90 degrees, but also a substantially perpendicular case of a slightly inclined puncture of the surface of the target site 100 for the purpose of injecting a medicinal solution into the target site 100.

A thickness dimension of the target site 100 such as an intramucosal or intradermal site varies depending on specific tissue, but in the present invention, a site having a size of more than 0.1 mm and 2.0 mm or less is an application target.

The injection needle 11 includes: in general, a bevel-needle-shaped needle body 30 having a hollow portion 31 through which a medicinal solution is passable; a needle body blade surface 32 which is formed at a distal portion of the needle body 30 and enables a puncture of the target site 100; and a passage hole 33 which is formed at the distal portion of the needle body 30 and through which the medicinal solution from the hollow portion 31 passes. The passage hole 33 is disposed biasedly toward a side where a distal end 34 of the needle body blade surface 32 is positioned. An edge of an opening edge of the passage hole 33 on a side opposite to the biased side is referred to as an "edge portion 35". A length L of the needle body 30 in an axial direction from the edge portion 35 to the distal end 34 of the needle body blade surface 32 is set to 0.1 mm or longer and shorter than 2.0 mm.

In the present specification, "to be disposed biasedly" means that, when a certain element is disposed in a certain member, the element is disposed eccentrically at a radially outward position with respect to an axial center of the member. When the above-described passage hole 33 is described as an example, the passage hole 33 is disposed eccentrically at a radially outward position with respect to an axial center of the needle body 30. In particular, the passage hole 33 is eccentric on a side on which the distal end 34 of the needle body blade surface 32 is positioned.

The injection needle 11 can puncture the target site 100 by the needle body blade surface 32 formed at the distal portion of the needle body 30 without increasing puncture resistance. The passage hole 33 is disposed biasedly toward the side on which the distal end 34 of the needle body blade surface 32 is positioned. The length L of the needle body 30 in the axial direction from the edge portion 35 of the passage hole 33 to the distal end 34 of the needle body blade surface 32 is set to 0.1 mm or longer and shorter than 2.0 mm. As compared with the case where the passage hole 33 is disposed at a position of the axial center of the bevel-needle-shaped needle body 30, it is possible to reduce a likelihood that the distal end 34 of the needle body blade surface 32 will pierce beyond the target site 100 into underlying tissue 101 under the target site 100. This makes it possible to prevent the medicinal solution from leaking into the underlying tissue 101. In addition, it is possible to reduce a likelihood that the passage hole 33 will be positioned above the target site 100. This makes it possible to prevent the medicinal solution from leaking to the surface of the target site 100. Further, since the needle body 30 with the needle body blade surface 32 having a relatively long length (bevel length) can be used, the puncture resistance does not increase, and it is possible to reliably achieve a puncture of the target site 100.

Hence, the injection needle 11 can puncture the target site 100 having a thickness dimension of more than 0.1 mm and 2.0 mm or less without increasing puncture resistance and can inject the medicinal solution into the target site 100 without causing a leakage of the medicinal solution.

An angle of the distal end 34 of the needle body blade surface 32 is preferably 30° or smaller. This configuration enables the puncture of the target site 100 without increasing the puncture resistance. The angle of the distal end 34 of the needle body blade surface 32 is preferably 10° or larger. A lower limit of the angle that can be obtained by grinding processing is about 5°, but a practical lower limit is 10° from the viewpoint of ease of grinding processing and mass productivity.

An outer diameter of the needle body 30 is set to at least 0.20 mm or larger. The smaller the outer diameter of the needle body 30, the more difficult it is to grind the distal end 34 of the needle body blade surface 32 to an acute angle. Therefore, the distal end 34 of the needle body blade surface 32 has a relatively obtuse angle, and the puncture resistance increases. When the needle body 30 has an outer diameter of 0.20 mm or larger, it is easy to grind the distal end 34 of the needle body blade surface 32 to an acute angle. Hence, the injection needle 11 can puncture the target site 100 without increasing the puncture resistance.

The injection needle 11 of Embodiment 1 will be described in more detail. As also illustrated in FIGS. 3A, 3B, 4A, and 4B, the injection needle 11 has a double-tube needle structure. The needle body 30 includes a first bevel needle 40 having a first lumen 41 and a first blade surface 42, and a second bevel needle 50 that has a second lumen 51 and a second blade surface 52 and is accommodated in the first lumen 41 of the first bevel needle 40.

The hollow portion 31 described above is formed by the second lumen 51 of the second bevel needle 50. The needle body blade surface 32 described above is formed by the first blade surface 42 of the first bevel needle 40. The passage hole 33 described above is formed by a discharge outlet 54 of the second lumen 51 of the second bevel needle 50. The axial center of the needle body 30 described above corresponds to an axial center of the first bevel needle 40, and the axial direction of the needle body 30 corresponds to an axial direction of the first bevel needle 40.

The second bevel needle 50 has the second blade surface 52 that is oriented in the same direction as the first blade surface 42 of the first bevel needle 40. The second bevel needle 50 is further disposed biasedly toward a side on which the distal end 43 of the first blade surface 42 of the first bevel needle 40 is positioned. The disposition position of the second bevel needle 50 is rephrased as follows. The second bevel needle 50 is disposed eccentrically outward in the radial direction with respect to the axial center of the first bevel needle 40, on the side on which the distal end 43 of the first blade surface 42 of the first bevel needle 40 is positioned.

The first lumen 41 of the first bevel needle 40 is sealed in a state where the second bevel needle 50 is disposed in the first lumen and does not allow a solution to pass. After the second bevel needle 50 is disposed in the first bevel needle 40, a sealing material 44 (for example, a resin material) is injected and fills the first lumen 41 to seal the first lumen 41. A portion of the sealing material 44 beyond the first blade surface 42 of the first bevel needle 40 is removed. Hence, as illustrated in FIG. 2, a distal portion of the sealing material 44 is not located beyond the first blade surface 42 of the first bevel needle 40.

As illustrated in FIGS. 2 and 4B, a distal end 53 of the second blade surface 52 of the second bevel needle 50 is not located beyond the first blade surface 42 of the first bevel needle 40. When the injection needle 11 punctures the target site 100, the first blade surface 42 of the first bevel needle 40 comes into contact with the target site 100 before the second blade surface 52 of the second bevel needle 50. The injection needle 11 punctures the target site 100 by the first blade surface 42 of the first bevel needle 40.

As illustrated in FIG. 2, the edge portion 35 is an edge of an opening edge of the discharge outlet 54 of the second lumen 51 of the second bevel needle 50 on the side opposite to the biased side of the second bevel needle. The length L of the first bevel needle 40 in the axial direction from the edge portion 35 to the distal end 43 of the first blade surface 42 of the first bevel needle 40 is set to 0.1 mm or longer and shorter than 2.0 mm.

As a material of the first bevel needle 40 and the second bevel needle 50, for example, stainless steel, aluminum, an aluminum alloy, titanium, a titanium alloy, or the like is used. It is preferable that the first bevel needle 40 have low puncture resistance. For example, it is preferable to reduce the puncture resistance by coating the surface of the first bevel needle 40 with a coating agent made of silicone resin, fluorine resin, or the like.

The injection needle 11 having the above-described configuration can puncture the target site 100 having a thickness dimension of more than 0.1 mm and 2.0 mm or less without increasing the puncture resistance and can inject the medicinal solution into the target site 100 without causing a leakage of the medicinal solution. In addition, the injection needle 11 can be manufactured by combining the existing first bevel needle 40 and the existing second bevel needle 50. Hence, the injection needle 11 can be manufactured relatively easily and inexpensively.

The second bevel needle 50 is disposed to be in contact with an inner circumferential surface 41a of the first lumen 41 of the first bevel needle 40. The discharge outlet 54 can be disposed most eccentrically, and it is easy to position the second bevel needle 50 with respect to the first bevel needle 40. In the case of this disposition, it is preferable that the first bevel needle 40 have a thin wall thickness. This is because the discharge outlet 54 can be disposed further eccentrically.

The angle of the distal end 43 of the first blade surface 42 of the first bevel needle 40 is more acute than an angle of the distal end 53 of the second blade surface 52 of the second bevel needle 50. Since an outer diameter of the first bevel needle 40 is larger than an outer diameter of the second bevel needle 50, it is easy to grind the distal end 43 of the first blade surface 42 to an acute angle. Since the injection needle 11 punctures the target site 100 by the first blade surface 42 of the first bevel needle 40, the puncture resistance of the injection needle 11 does not increase even when the second bevel needle 50 having the small outer diameter is used. Hence, the second bevel needle 50 having the small outer diameter can be used. As a result, the discharge outlet 54 can be disposed further eccentrically.

An example of a combination of the first bevel needle 40 and the second bevel needle 50 is as follows. The first bevel needle 40 has a 27G (outer diameter of 0.40 mm) needle tube having an inner diameter of 0.22 mm and a bevel length of the first blade surface 42 of 1.85 mm. The second bevel needle 50 has a 36G (outer diameter of 0.10 mm) needle tube having an inner diameter of 0.04 mm and a bevel length of the second blade surface 52 of 0.15 mm. In this combination, the length L is about 0.3 mm. According to this combination, it is possible to puncture the target site 100 having a thickness dimension of more than 0.1 mm and 2.0 mm or less without increasing puncture resistance and inject the medicinal solution into the target site 100 without causing a leakage of the medicinal solution.

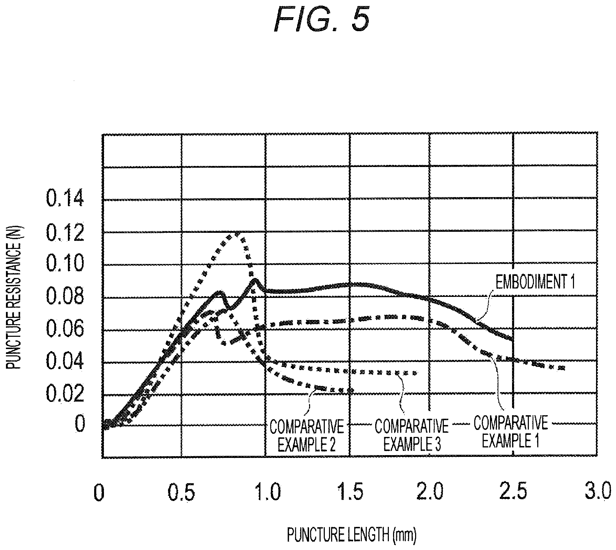

FIG. 5 is a graph illustrating an experimental result of the puncture resistance of the injection needle 11.

In an experiment, Autograph (EZ-L) manufactured by Shimadzu Corporation is used, a syringe fixing jig is set in the Autograph, and a silicone sheet is lowered so that the injection needle 11 punctures the silicone sheet. The resistance when the injection needle 11 punctured the silicone sheet was measured and recorded as the puncture resistance of the injection needle 11.

The injection needle 11 of Embodiment 1 is a combination of the first bevel needle 40 and the second bevel needle 50 described above. A needle tube of Comparative Example 1 is 27G (outer diameter of 0.40 mm) and has a bevel length of 1.85 mm. A needle tube of Comparative Example 2 is 36G (outer diameter of 0.10 mm) and has a bevel length of 0.52 mm. A needle tube of Comparative Example 3 is 34G (outer diameter of 0.20 mm) and has a bevel length of 0.3 mm.

As illustrated in FIG. 5, the injection needle 11 of Embodiment 1 had the puncture resistance of about 0.082 N. The needle tube of Comparative Example 1 had the puncture resistance of about 0.065 N. The needle tube of Comparative Example 2 had the puncture resistance of about 0.073 N. The needle tube of Comparative Example 3 had the puncture resistance of about 0.114 N.

The first bevel needle 40 of Embodiment 1 uses the 27G needle tube, but the puncture resistance was slightly higher than that of the needle tube of Comparative Example 1. The reason is presumed to be a processing problem. That is, it is assumed that, in Embodiment 1, the sealing material 44 partially adhered to the first blade surface 42 of the first bevel needle 40, and this causes the puncture resistance to be higher than that of Comparative Example 1. The injection needle 11 of Embodiment 1 had the same puncture resistance as the needle tube of Comparative Example 2 (36G and the bevel length of 0.52 mm). The injection needle 11 of Embodiment 1 had the puncture resistance lower than that of the needle tube of Comparative Example 3 (34G and the bevel length of 0.3 mm).

From the results of the puncture resistance of the injection needle 11 of Embodiment 1 and the needle tubes of Comparative Examples 2 and 3, it was verified that the puncture resistance can be reduced by forming the double-tube structure with the first bevel needle 40 and the second bevel needle 50 even when the second bevel needle 50 having the small outer diameter and the short bevel length is used.

As described above, the injection needle 11 of Embodiment 1 is the injection needle 11 that perpendicularly punctures the surface of the target site 100 such as an intramucosal or intradermal site and injects the medicinal solution into the target site 100. The injection needle 11 includes: the bevel-needle-shaped needle body 30 having the hollow portion 31 through which the medicinal solution is passable; the needle body blade surface 32 which is formed at the distal portion of the needle body 30 and enables the puncture of the target site 100; and the passage hole 33 which is formed at the distal portion of the needle body 30 and through which the medicinal solution from the hollow portion 31 passes. The passage hole 33 is disposed biasedly toward a side where a distal end 34 of the needle body blade surface 32 is positioned. The length L of the needle body 30 in the axial direction from the edge portion 35 of the opening edge of the passage hole 33 on the side opposite to the biased side of the opening edge to the distal end 34 of the needle body blade surface 32 is set to 0.1 mm or longer and shorter than 2.0 mm.

The injection needle 11 having the configuration described above can puncture the target site 100 by the needle body blade surface 32 formed at the distal portion of the needle body 30 without increasing puncture resistance. The passage hole 33 is disposed biasedly toward the side on which the distal end 34 of the needle body blade surface 32 is positioned. The length L of the needle body 30 in the axial direction from the edge portion 35 of the passage hole 33 to the distal end 34 of the needle body blade surface 32 is set to 0.1 mm or longer and shorter than 2.0 mm. As compared with the case where the passage hole 33 is disposed at a position of the axial center of the bevel-needle-shaped needle body 30, it is possible to reduce a likelihood that the distal end 34 of the needle body blade surface 32 will pierce beyond the target site 100 into underlying tissue 101 under the target site 100. This makes it possible to prevent the medicinal solution from leaking into the underlying tissue 101. In addition, it is possible to reduce a likelihood that the passage hole 33 will be positioned above the target site 100. This makes it possible to prevent the medicinal solution from leaking to the surface of the target site 100. Further, since the needle body 30 with the needle body blade surface 32 having a relatively long length (bevel length) can be used, the puncture resistance does not increase, and it is possible to reliably achieve a puncture of the target site 100. Therefore, the injection needle 11 can puncture the target site 100 having a thickness dimension of more than 0.1 mm and 2.0 mm or less without increasing puncture resistance and can inject the medicinal solution into the target site 100 without causing a leakage of the medicinal solution.

The angle of the distal end 34 of the needle body blade surface 32 is 30° or smaller. This configuration enables the puncture of the target site 100 without increasing the puncture resistance.

An outer diameter of the needle body 30 is set to at least 0.20 mm or larger. This configuration makes it easy to grind the distal end 34 of the needle body blade surface 32 to an acute angle. Hence, the injection needle 11 can puncture the target site 100 without increasing the puncture resistance.

The needle body 30 includes a first bevel needle 40 having a first lumen 41 and a first blade surface 42, and a second bevel needle 50 that has a second lumen 51 and a second blade surface 52 and is accommodated in the first lumen 41 of the first bevel needle 40. The hollow portion 31 is formed by the second lumen 51 of the second bevel needle 50, the needle body blade surface 32 is formed by the first blade surface 42 of the first bevel needle 40, and the passage hole 33 is formed by the discharge outlet 54 of the second lumen 51 of the second bevel needle 50. The second bevel needle 50 has the second blade surface 52 that is oriented in the same direction as the first blade surface 42 of the first bevel needle 40 and is disposed biasedly toward the side on which the distal end 43 of the first blade surface 42 of the first bevel needle 40 is positioned. The first lumen 41 of the first bevel needle 40 is sealed in a state where the second bevel needle 50 is disposed therein, and the distal end 53 of the second blade surface 52 of the second bevel needle 50 is not located beyond the first blade surface 42 of the first bevel needle 40. This configuration enables the injection needle 11 to puncture the target site 100 having a thickness dimension of more than 0.1 mm and 2.0 mm or less without increasing the puncture resistance and to inject the medicinal solution into the target site 100 without causing a leakage of the medicinal solution. In addition, the injection needle 11 can be manufactured by combining the existing first bevel needle 40 and the existing second bevel needle 50. Hence, the injection needle 11 can be manufactured relatively easily.

The second bevel needle 50 is disposed to be in contact with an inner circumferential surface 41a of the first lumen 41 of the first bevel needle 40. This configuration enables the discharge outlet 54 to be disposed most eccentrically and makes it easy to position the second bevel needle 50 with respect to the first bevel needle 40.

The angle of the distal end 43 of the first blade surface 42 of the first bevel needle 40 is more acute than an angle of the distal end 53 of the second blade surface 52 of the second bevel needle 50. According to this configuration, since an outer diameter of the first bevel needle 40 is larger than an outer diameter of the second bevel needle 50, it is easy to grind the distal end 43 of the first blade surface 42 to an acute angle. Since the injection needle 11 punctures the target site 100 by the first blade surface 42 of the first bevel needle 40, the puncture resistance of the injection needle 11 does not increase even when the second bevel needle 50 having the small outer diameter is used. Hence, the second bevel needle 50 having the small outer diameter can be used. As a result, the discharge outlet 54 can be disposed further eccentrically.

The first bevel needle 40 has a 27G (outer diameter of 0.40 mm) needle tube having an inner diameter of 0.22 mm and a bevel length of the first blade surface 42 of 1.85 mm. The second bevel needle 50 has a 36G (outer diameter of 0.10 mm) needle tube having an inner diameter of 0.04 mm and a bevel length of the second blade surface 52 of 0.15 mm. According to this combination, it is possible to puncture the target site 100 having a thickness dimension of more than 0.1 mm and 2.0 mm or less without increasing puncture resistance and inject the medicinal solution into the target site 100 without causing a leakage of the medicinal solution.

In Embodiment 1, the 27G (outer diameter of 0.40 mm) needle tube is used as the first bevel needle 40. However, it is possible to attach the second bevel needle 50 by using the 34G (the outer diameter of 0.20 mm, the inner diameter of 0.16 mm, and a wall thickness of 0.02 mm) needle tube as the first bevel needle 40. However, a degree of bias of the discharge outlet 54 of the second bevel needle 50 (a degree of eccentricity with respect to the axial center of the first bevel needle 40) decreases. In order to increase the degree of bias of the passage hole 33, the following modes from Embodiment 2 to be described below can be employed.

### (Embodiment 2)

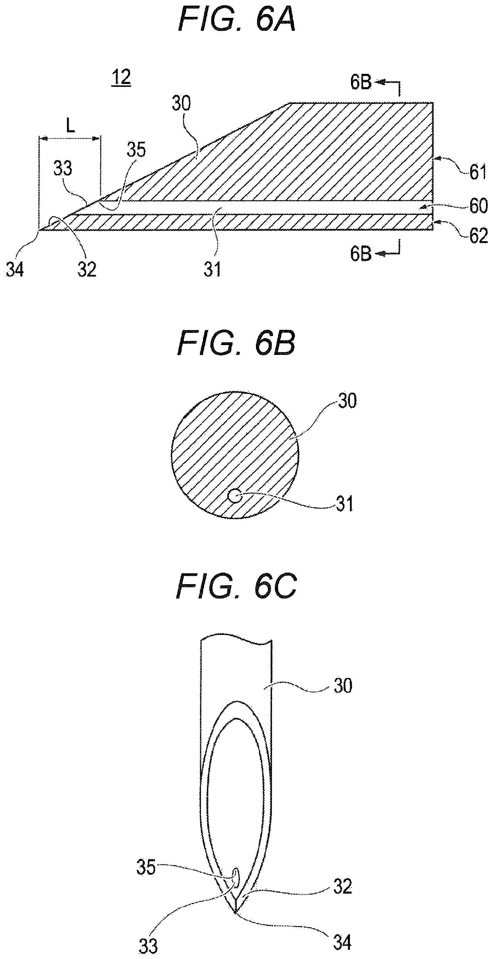

FIGS. 6A, 6B, and 6C are a longitudinal-sectional view illustrating an injection needle 12 of Embodiment 2, a cross-sectional view along line 6B-6B of FIG. 6A, and a top view of the injection needle.

The injection needle 12 of Embodiment 2 includes: in general, similarly to the injection needle 11 of Embodiment 1, a bevel-needle-shaped needle body 30 having a hollow portion 31 through which a medicinal solution is passable; a needle body blade surface 32 which is formed at a distal portion of the needle body 30 and enables a puncture of the target site 100; and a passage hole 33 which is formed at the distal portion of the needle body 30 and through which the medicinal solution from the hollow portion 31 passes. The passage hole 33 is disposed biasedly toward a side where a distal end 34 of the needle body blade surface 32 is positioned. The length L of the needle body in an axial direction from an edge portion 35 of an opening edge of the passage hole 33 on a side opposite to the biased side of the opening edge to the distal end 34 of the needle body blade surface 32 is set to 0.1 mm or longer and shorter than 2.0 mm.

The injection needle 12 of Embodiment 2 can also puncture the target site 100 having a thickness dimension of more than 0.1 mm and 2.0 mm or less without increasing the puncture resistance and can inject the medicinal solution into the target site 100 without causing a leakage of the medicinal solution.

In addition, similarly to the injection needle 11 of Embodiment 1, an angle of the distal end 34 of the needle body blade surface 32 is 30° or smaller. An outer diameter of the needle body 30 is set to at least 0.20 mm or larger.

In the injection needle 12 of Embodiment 2, the needle body 30 is formed of a metal material. As the metal material, for example, stainless steel, aluminum, an aluminum alloy, titanium, a titanium alloy, or the like is used.

The hollow portion 31 and the passage hole 33 described above are formed by a lumen 60 parallel to the axial direction of the needle body 30. The lumen 60 is formed by performing, for example, laser machining, electrical discharge machining, or the like on a solid metal material.

The needle body 30 has a thick region 61 (upper region on the lumen 60 in FIG. 6A) having a thick wall thickness and a thin region 62 (lower region under the lumen 60 in FIG. 6A) having a thin wall thickness, the thick region 61 and the thin region 62 being formed by disposing the lumen 60 biasedly. The wall thickness in the thin region 62 is set to at least 0.02 mm or more.

The injection needle 12 of Embodiment 2 has an uneven-thickness needle structure in which the thick region 61 and the thin region 62 are formed. It is possible to dispose the lumen 60 at the axial center and form a needle tube having a small outer diameter, but it is not possible to secure the needle strength suitable for the puncturing. In addition, the blade surface cannot be formed by performing grinding. The blade surface can be formed by electroforming or electric discharge machining, but the puncture resistance can be reduced by forming the blade surface by performing grinding. In this respect, when the injection needle 12 has the uneven-thickness needle structure, it is possible to secure the needle strength suitable for the puncturing. In addition, since the needle body blade surface 32 can be formed by grinding, the injection needle 12 having low puncture resistance can be formed.

The reason why the wall thickness in the thin region 62 is set to at least 0.02 mm or more is to secure the needle strength suitable for the puncturing and to enable the needle body blade surface 32 to be formed by performing grinding.

As described above, in the injection needle 12 of Embodiment 2, the needle body 30 is made of a metal material, and the hollow portion 31 and the passage hole 33 are formed by the lumen 60 parallel to the axial direction of the needle body 30. The needle body 30 has the thick region 61 having the thick wall thickness and the thin region 62 having the thin wall thickness, the thick region 61 and the thin region 62 being formed by disposing the lumen 60 biasedly. The wall thickness in the thin region 62 is set to at least 0.02 mm or more. The injection needle 12 of Embodiment 2 can also puncture the target site 100 having a thickness dimension of more than 0.1 mm and 2.0 mm or less without increasing the puncture resistance and can inject the medicinal solution into the target site 100 without causing a leakage of the medicinal solution. Further, it is possible to secure the needle strength suitable for the puncturing, and since the needle body blade surface 32 can be formed by performing grinding, the injection needle 12 having the low puncture resistance can be formed.

### (Embodiment 3)

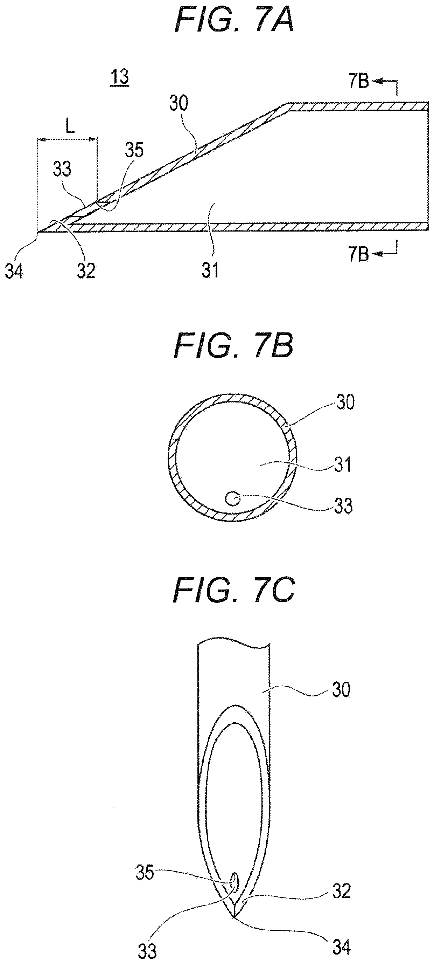

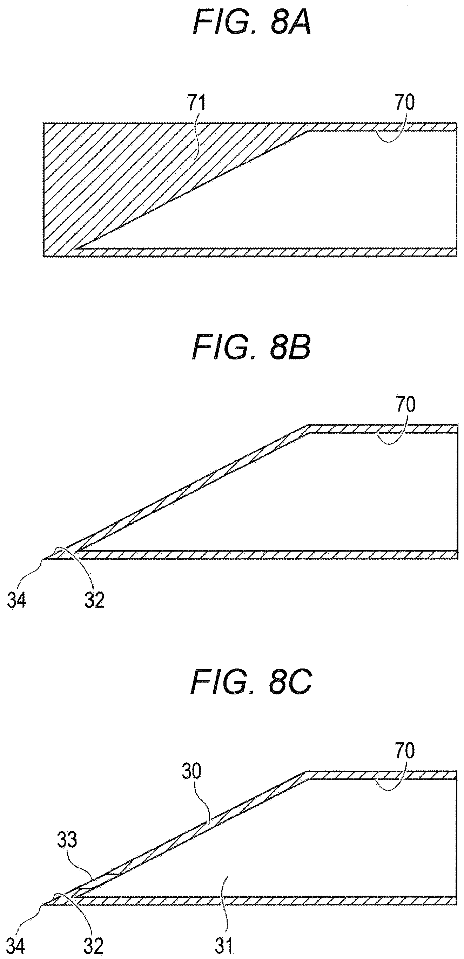

FIGS. 7A, 7B, and 7C are a longitudinal-sectional view illustrating an injection needle 13 of Embodiment 3, a cross-sectional view along line 7B-7B of FIG. 7A, and a top view of the injection needle. FIGS. 8A, 8B, and 8C are schematic views illustrating a procedure for forming the injection needle 13 of Embodiment 3. The same members as those of the injection needles 11 and 12 of Embodiments 1 and 2 are denoted by the same reference numerals, and the description thereof will be partially omitted.

In the injection needle 13 of Embodiment 3, the needle body 30 is formed of a metal material. As the metal material, for example, stainless steel, aluminum, an aluminum alloy, titanium, a titanium alloy, or the like is used.

The hollow portion 31 described above is formed by a lumen 70 concentric to an axial center of the needle body 30. A wall thickness of the needle body 30 is set to at least 0.20 mm or larger.

In the case of forming the injection needle 13 of Embodiment 3, first, as illustrated in FIG. 8A, a pipe 71 having the lumen 70 is manufactured by electroforming.

Next, as illustrated in FIG. 8B, the needle body blade surface 32 is formed by performing grinding.

Then, as illustrated in FIG. 8C, the passage hole 33 is formed at the distal portion of the needle body 30 by electrical discharge machining or laser machining.

Similarly to the injection needle 12 of Embodiment 2, the reason why the wall thickness of the needle body 30 is set to at least 0.02 mm or more is to secure the needle strength suitable for the puncturing and to enable the needle body blade surface 32 to be formed by performing grinding.

As described above, in the injection needle 13 of Embodiment 3, the needle body 30 is made of a metal material, and the hollow portion 31 is formed by the lumen 70 concentric to the axial center of the needle body 30. A wall thickness of the needle body 30 is set to at least 0.20 mm or larger. The injection needle 13 of Embodiment 3 can also puncture the target site 100 having the thickness dimension of more than 0.1 mm and 2.0 mm or less without increasing the puncture resistance and can inject the medicinal solution into the target site 100 without causing a leakage of the medicinal solution. Further, it is possible to secure the needle strength suitable for the puncturing, and since the needle body blade surface 32 can be formed by performing grinding, the injection needle 12 having the low puncture resistance can be formed.

### (Embodiment 4)

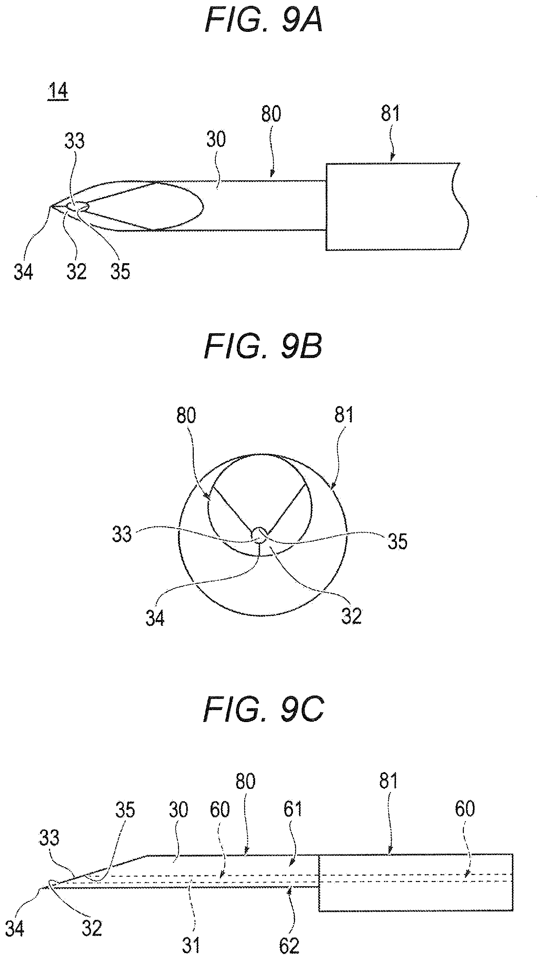

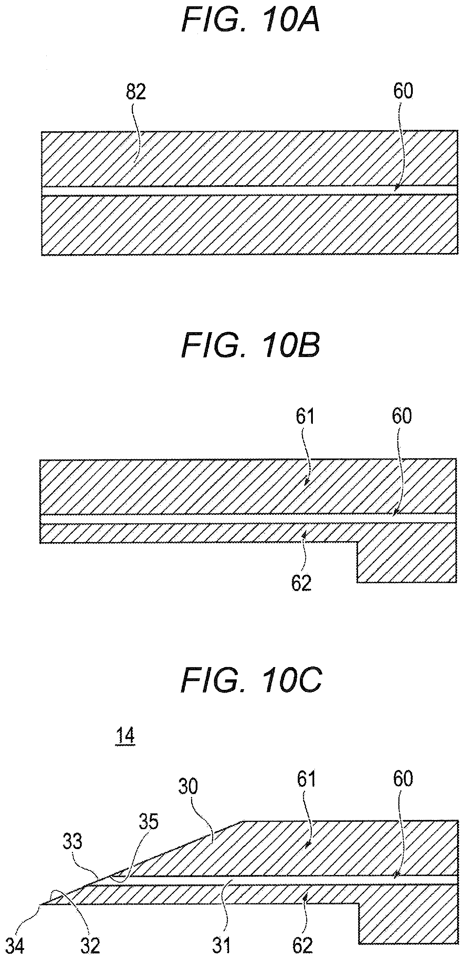

FIGS. 9A, 9B, and 9C are a top view, a front view, and a side view illustrating an injection needle 14 of Embodiment 4. FIGS. 10A, 10B, and 10C are schematic views illustrating a procedure for forming the injection needle 14 of Embodiment 4. The same members as those of the injection needles 11 to 13 of Embodiments 1 to 3 are denoted by the same reference numerals, and the description thereof will be partially omitted.

The injection needle 14 of Embodiment 4 has a small-diameter portion 80 on the distal end side and a large-diameter portion 81 on the proximal end side.

The small-diameter portion 80 of the injection needle 14 is configured similarly to the injection needle 12 of Embodiment 2. The needle body 30 has a thick region 61 (upper region on a lumen 60 in FIG. 9C) having a thick wall thickness and a thin region 62 (lower region under the lumen 60 in FIG. 9C) having a thin wall thickness, the thick region 61 and the thin region 62 being formed by disposing the lumen 60 biasedly. The wall thickness in the thin region 62 is set to at least 0.02 mm or more.

In the large-diameter portion 81 of the injection needle 14, the lumen 60 is disposed close to the axial center.

In the case of forming the injection needle 14 of Embodiment 4, first, as illustrated in FIG. 10A, a thick pipe 82 in which the lumen 60 is formed at the axial center is prepared.

Next, as illustrated in FIG. 10B, a distal end side of the thick pipe 82 on one side (side below the lumen 60) is scraped by electrical discharge machining or polishing to form the thin region 62.

Next, as illustrated in FIG. 10C, the needle body blade surface 32 is formed by performing grinding.

The reason why the wall thickness in the thin region 62 is set to at least 0.02 mm or more is to secure the needle strength suitable for the puncturing and to enable the needle body blade surface 32 to be formed by performing grinding.

The injection needle 14 of Embodiment 4 can also puncture the target site 100 having the thickness dimension of more than 0.1 mm and 2.0 mm or less without increasing the puncture resistance and can inject the medicinal solution into the target site 100 without causing a leakage of the medicinal solution. Further, it is possible to secure the needle strength suitable for the puncturing, and since the needle body blade surface 32 can be formed by performing grinding, the injection needle 12 having the low puncture resistance can be formed.

### (Embodiment 5)

Embodiment 5 is a modification of the procedure for forming an injection needle 15 having an uneven-thickness needle structure (the same structure as the injection needle 12 of Embodiment 2).

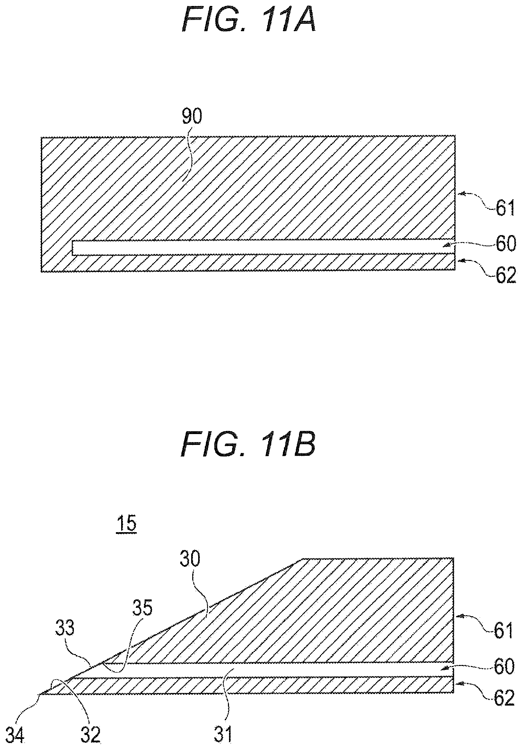

FIGS. 11A and 11B are schematic views illustrating a procedure for forming the injection needle 15 of Embodiment 5.

First, as illustrated in FIG. 11A, a pipe 90 in which a lumen 60 is disposed biasedly is formed by electroforming. The lumen 60 does not penetrate the pipe 90.

Then, as illustrated in FIG. 11B, a needle body blade surface 32 is formed by performing grinding until the lumen 60 appears.

The injection needle 15 of Embodiment 5 can also puncture the target site 100 having the thickness dimension of more than 0.1 mm and 2.0 mm or less without increasing the puncture resistance and can inject the medicinal solution into the target site 100 without causing a leakage of the medicinal solution. Further, it is possible to secure the needle strength suitable for the puncturing, and since the needle body blade surface 32 can be formed by performing grinding, the injection needle 12 having the low puncture resistance can be formed.

Although the injection needles 11 to 15 according to the present invention have been described above through Embodiments 1 to 5, the present invention is not limited only to the contents described in the embodiment, and can be appropriately changed on the basis of the description in the claims.

For example, in Embodiment 1, the angle of the distal end 43 of the first blade surface 42 of the first bevel needle 40 is more acute than the angle of the distal end 53 of the second blade surface 52 of the second bevel needle 50. However, the present invention is not limited to this case. The angle of the distal end 43 of the first blade surface 42 and the angle of the distal end 53 of the second blade surface 52 can be set to the same angle, and the first blade surface 42 and the second blade surface 52 can be aligned.

The present application is based on Japanese Patent Application No. 2021-165236 filed on October 7, 2021, the disclosure content of which is incorporated herein by reference in its entirety.

### Reference Signs List

- 11-15: injection needle
- 20: injection needle assembly
- 21: hub
- 30: needle body
- 31: hollow portion
- 32: needle body blade surface
- 33: passage hole
- 34: distal end
- 35: edge portion
- 40: first bevel needle
- 41: first lumen
- 41a: inner circumferential surface
- 42: first blade surface
- 43: distal end
- 44: sealing material
- 50: second bevel needle
- 51: second lumen
- 52: second blade surface
- 53: distal end
- 54: discharge outlet
- 60: lumen
- 61: thick region
- 62: thin region
- 70: lumen
- 71: pipe
- 80: small-diameter portion
- 81: large-diameter portion
- 82: thick pipe
- 90: pipe
- 100: target site
- 101: underlying tissue

## Claims

1. An injection needle that perpendicularly punctures a surface of a target site such as an intramucosal or intradermal site and injects a medicinal solution into the target site, the injection needle comprising:
a bevel-needle-shaped needle body having a hollow portion through which the medicinal solution is passable;
a needle body blade surface which is formed at a distal portion of the needle body and enables a puncture of the target site; and
a passage hole which is formed at the distal portion of the needle body and through which the medicinal solution from the hollow portion passes, wherein
the passage hole is disposed biasedly toward a side on which a distal end of the needle body blade surface is positioned, and
a length in an axial direction of the needle body from an edge portion of an opening edge of the passage hole on a side opposite to a biased side of the opening edge to the distal end of the needle body blade surface is set to 0.1 mm or longer and shorter than 2.0 mm.

2. The injection needle according to claim 1, wherein the distal end of the needle body blade surface has an angle of 30° or smaller.

3. The injection needle according to claim 1 or 2, wherein the needle body has an outer diameter of at least 0.20 mm or larger.

4. The injection needle according to any one of claims 1 to 3, wherein
the needle body includes a first bevel needle having a first lumen and a first blade surface, and a second bevel needle that has a second lumen and a second blade surface and is accommodated in the first lumen of the first bevel needle,
the hollow portion is formed by the second lumen of the second bevel needle,
the needle body blade surface is formed by the first blade surface of the first bevel needle,
the passage hole is formed by a discharge outlet of the second lumen of the second bevel needle,
the second bevel needle has the second blade surface that is oriented in the same direction as the first blade surface of the first bevel needle and is disposed biasedly toward a side on which a distal end of the first blade surface of the first bevel needle is positioned,
the first lumen of the first bevel needle is sealed in a state where the second bevel needle is disposed in the first lumen, and
a distal end of the second blade surface of the second bevel needle is not disposed beyond the first blade surface of the first bevel needle.

5. The injection needle according to claim 4, wherein the second bevel needle is disposed in contact with an inner circumferential surface of the first lumen of the first bevel needle.

6. The injection needle according to claim 4 or 5, wherein an angle of the distal end of the first blade surface of the first bevel needle is more acute than an angle of the distal end of the second blade surface of the second bevel needle.

7. The injection needle according to any one of claims 4 to 6, wherein
the first bevel needle has a 27G (outer diameter of 0.40 mm) needle tube having an inner diameter of 0.22 mm and a bevel length of the first blade surface of 1.85 mm, and
the second bevel needle has a 36G (outer diameter of 0.10 mm) needle tube having an inner diameter of 0.04 mm and a bevel length of the second blade surface of 0.15 mm.

8. The injection needle according to any one of claims 1 to 3, wherein
the needle body is made of a metal material,
the hollow portion and the passage hole are formed by a lumen parallel to the axial direction of the needle body,
the needle body has a thick region having a thick wall and a thin region having a thin wall, the thick region and the thin region being formed by disposing the lumen biasedly, and
a wall thickness in the thin region is set to at least 0.02 mm or more.

9. The injection needle according to any one of claims 1 to 3, wherein
the needle body is made of a metal material,
the hollow portion is formed by a lumen concentric to an axial center of the needle body, and
a wall thickness of the needle body is set to at least 0.02 mm or more.
